# EUROPEAN PATENT SPECIFICATION

(11) **EP 1 920 740 B1**
(45) Date of publication and mention of the grant of the patent: **07.03.2012**
(21) Application number: 07016557.6
(22) Date of filing: 23.08.2007
(51) Int. Cl.: A61F 2/88, A61F 2/84

(54) **Stent supply instrument**
Instrument zum Einführen eines Stents
Instrument d'approvisionnement d'endoprothèse vasculaire

(30) Priority: 09.11.2006 JP 2006303698
(43) Date of publication of application: 14.05.2008
(73) Proprietor: Olympus Medical Systems Corp., Tokyo 151-0072 (JP)
(72) Inventor: Goto, Hiroaki, Shibuya-ku Tokyo 151-0072 (JP)
(74) Representative: von Hellfeld, Axel

(56) References cited:
- EP-A- 0 631 762
- WO-A-94/16629
- WO-A-95/30385
- FR-A- 2 767 673

## Description

### BACKGROUND OF THE INVENTION

### Description of the Related Art

When a stent having an outer diameter larger than an outer diameter of an endoscope and including a metal wire formed spirally is oral-endoscopically inserted into a coelom, there has been suggested a stent supply instrument for drawing the stent in an axial direction to reduce a loop diameter and inserting the stent between an inner tube and an outer sheath to hold the diameter-reduced shape so as to make the insertion of the stent easy (see PCT Japanese Translation Patent Publication No. 2004-527297). In such a stent supply instrument, the stent can be supplied with a reduced diameter until the stent is detained in a desired position and the diameter of the stent can be expanded at the time of detaining the stent.

FR 2 767 673 discloses a urethral prosthesis having first through third sections, the first and third sections having a conical shape, and the second section disposed between the first and third section having a cylindrical shape adapted to hold open a urethra. The prosthesis is formed by winding a metallic wire into joined spiral coils over a mandrel. The prosthesis may be introduced in to an introduction tube having a smaller diameter than the largest diameter of the prosthesis to be placed in the urethra. Because the prosthesis is formed of a wire having elasticity, the prosthesis can be introduced in to the introduction tube by being elastically deformed in a radial direction within the tube so that the coils are displaced away from each other on one side, resulting in a smaller apparent diameter with respect to the introduction tube. The elastically deformed prosthesis is pushed from the introduction tube into the urethra by a pusher, which returns to its original form spontaneously after being ejected from the introduction tube.

However, contrary to a metal stent of which the loop diameter can be easily reduced and expanded due to its metal wire being wound, a resin stent has a restoring force smaller than that of the metal stent. Accordingly, even when the loop diameter of the resin stent is changed to supply the resin stent to a detainment position with the known stent supply instrument, it is difficult to restore the resin stent to the original shape. Therefore, it is not possible to utilize the known stent supply instrument.

The invention is made to solve the above-mentioned problem. An object of the invention is to provide a stent supply instrument which can easily supply a resin stent into a coelom.

### SUMMARY OF THE INVENTION

In order to accomplish the above-mentioned object, a stent supply instrument according to claim 1 is provided.

A stent supply instrument according to an aspect of the invention includes: a plurality of loops formed by spirally winding a wire or a strand wire made of a resin; joint portions that joint the loops to each other along a common center line; and a deformation holding section that holds a deformed shape of a stent detained in a coelom while holding the jointed state of the loops so that a projection area of the loops after deformation projected onto a virtual plane perpendicular to the center axis is smaller than the projection area of the loops before deformation projected onto the virtual plane. The stent is released from the deformation holding section by means of a relative movement between the deformation holding section and the stent.

By deforming the stent so that the projection area of the stent onto the virtual plane perpendicular to the center line of the stent is smaller than the projection area thereof before deformation, it is possible to insert the stent into a coelom with the stent reduced to a diameter smaller than the original outer diameter thereof. At this time, since the jointed state of the loops is held, it is possible to restore the shape of the stent by means of a relative movement between the stent and the deformation holding section.

In the stent supply instrument, the deformation holding section may be formed in a cylindrical shape with an inner diameter smaller than an outer diameter of the stent, and the stent supply instrument may further include a release portion disposed in the deformation holding section so as to advance and retreat and to come in contact with the stent of which the deformed state is held by the deformation holding section.

By moving the release portion toward the distal end of the deformation holding section relative to the stent, it is possible to extrude the stent disposed in the deformation holding section toward the distal end of the deformation holding section and to detain the stent.

In the stent supply instrument, the deformation holding section may include an inner diameter holding portion for suppressing the stent from being additionally deformed so as to reduce a projection area of the stent after deformation onto the virtual plane and an outer diameter holding portion for pressing the stent after deformation against the inner diameter holding portion.

Accordingly, it is possible to suppress the stent from being further reduced in diameter by the use of the inner diameter holding portion. On the other hand, it is possible to properly hold the deformed state of the stent by the use of the outer diameter holding portion. By releasing the pressing of the outer diameter holding portion against the inner diameter holding portion, it is possible to restore the shape of the stent.

In the stent supply instrument, the outer diameter holding portion may be formed in a line shape and be wound on the stent.

By winding the outer diameter holding portion on the stent, it is possible to bring the stent into close contact with the inner diameter holding portion. By removing the outer diameter holding portion from the stent, it is possible to restore the shape of the stent.

In the stent supply instrument, the outer diameter holding portion may be formed in a tube shape with an inner diameter smaller than the outer diameter of the stent before deformation and equal to or greater than the outer diameter of the loops after deformation projected onto the virtual plane.

By inserting the inner diameter holding portion into the outer diameter holding portion along with the deformed stent disposed on the outer peripheral surface of the inner diameter holding portion, it is possible to properly hold the deformed state of the stent.

In the stent supply instrument, the deformation holding section may hold the deformed stent with an inner diameter greater than the outer diameter of an insertion section of an endoscope and may allow the insertion section to pass through the stent so as to freely advance and retreat.

It is possible to visually recognize the supply and detainment states of the stent by the use of the insertion section of the endoscope.

In the stent supply instrument, the deformation holding section may be formed in a cylindrical shape with an outer diameter smaller than the outer diameter of the stent, and a plurality of slits extending in a center line direction may be formed at the distal end of the deformation holding section.

By inserting a part of the stent into the slits, a part of the side surface of the stent is disposed along the outer peripheral surface of the deformation holding section and the other part thereof is disposed along the inner peripheral surface of the deformation holding section. As a result, since the stent can be deformed relative to the outer periphery of the deformation holding section, it is possible to allow the projection area of the loops after deformation onto the virtual plane to be smaller than the projection area thereof before deformation.

According to the invention, it is possible to easily supply a resin stent into a coelom.

### BRIEF DESCRIPTION OF THE DRAWINGS

FIG. 1 is a perspective view illustrating a stent which is detained by the use of a stent supply instrument according to a first embodiment of the invention.
FIG. 2 is a partial perspective view illustrating the stent supply instrument according to the first embodiment of the invention.
FIG. 3 is a perspective view illustrating a deformed state of the stent at the time of supplying the stent by the use of the stent supply instrument according to the first embodiment of the invention.
FIG. 4 is a perspective view illustrating a deformed state of the stent at the time of supplying the stent by the use of the stent supply instrument according to the first embodiment of the invention.
FIG. 5 is a comparative diagram illustrating states of the stent before and after deformation at the time of supplying the stent by the use of the stent supply instrument according to the first embodiment of the invention.
FIG. 6 is a comparative diagram illustrating states of the stent before and after deformation at the time of supplying the stent by the use of the stent supply instrument according to the first embodiment of the invention.
FIG. 7 is a partial perspective view illustrating a stent supply instrument according to a second embodiment of the invention.
FIG. 8 is a partial perspective view illustrating a stent supply instrument according to a third embodiment of the invention.
FIG. 9 is a partial perspective view illustrating a stent supply instrument according to a fourth embodiment of the invention.
FIG. 10A and FIG. 10B are partial perspective views illustrating a stent supply instrument according to a fifth embodiment of the invention.
FIG. 11 is a partial perspective view illustrating a modified example of the stent supply instrument according to the second embodiment of the invention.
FIG. 12 is a partial perspective view illustrating a modified example of the stent supply instrument according to the third embodiment of the invention.

### DETAILED DESCRIPTION OF THE INVENTION

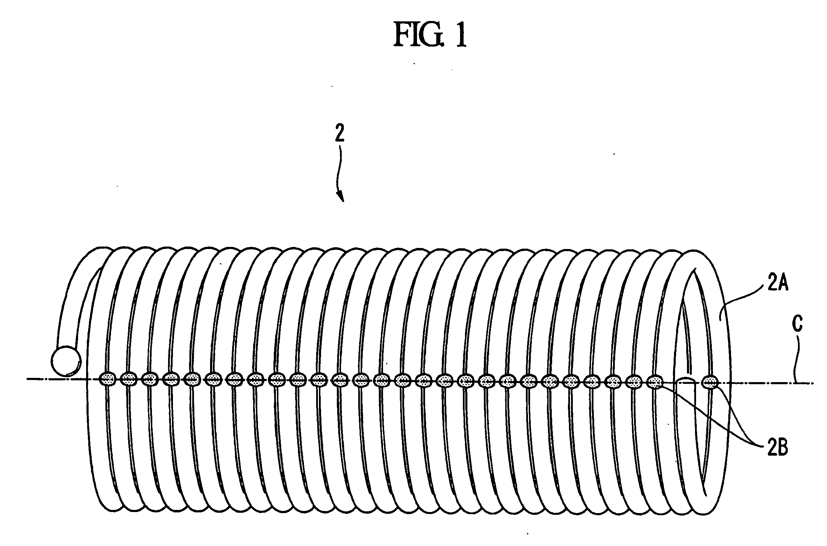

A first embodiment of the invention will be described with reference to FIGS. 1 to 6.

As shown in FIG. 1, a stent supply instrument 1 according to the first embodiment is designed to detain in a coelom a stent 2 having a plurality of ring-shaped loops 2A formed by spirally winding a wire or a strand wire made of a resin and joint portions 2B that joint the loops 2A to each other along a common center line C. The joint portions 2B bond or weld the neighboring loops 2A to each other at one or two or more positions (two positions in this embodiment).

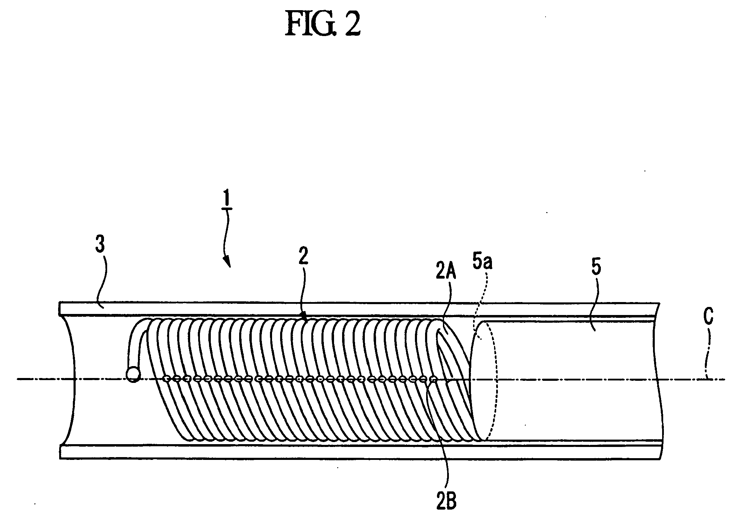

As shown in FIG. 2, the stent supply instrument 1 includes an over tube (deformation holding section) 3 which has a tube shape and which holds a deformed state of the stent 2 inserted therein in the deformed state and a rod-shaped extrusion member (release portion) 5 which is disposed in the over tube 3 so as to come in contact with an end of the stent 2 of which the deformed state is held by the over tube 3 and to freely advance and retreat.

The over tube 3 has an inner diameter (for example, 18 mm) smaller than the outer diameter of the stent 2 (for example, 20 mm). The extrusion member 5 has a rod shape and a distal end surface 5a thereof comes in contact with the stent 2.

Next, operations of the stent supply instrument 1 according to this embodiment will be described.

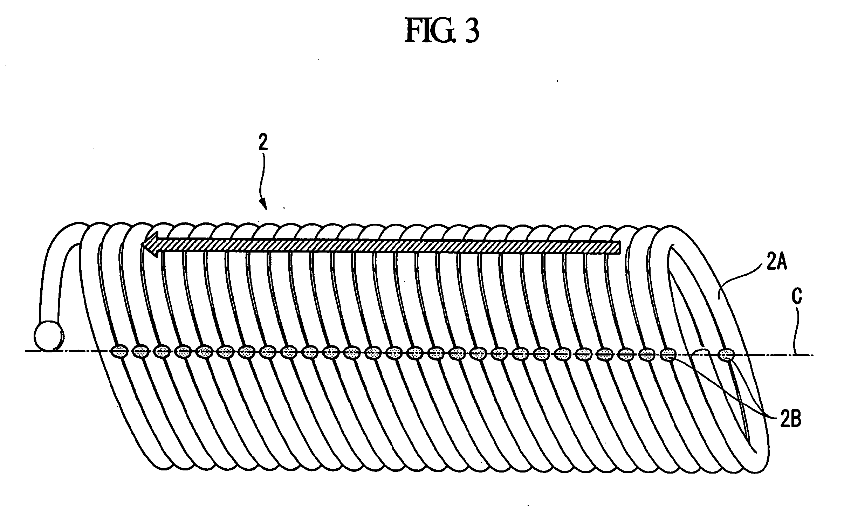

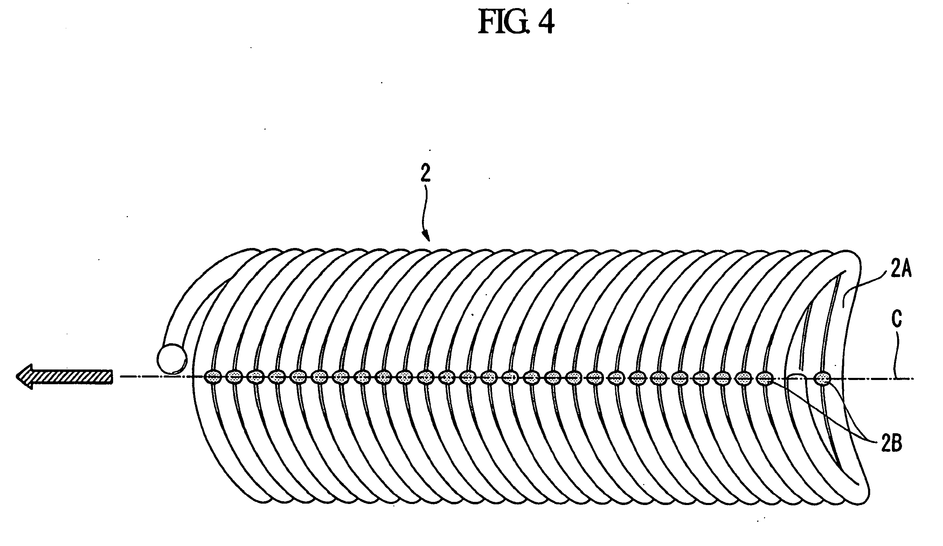

First, the stent 2 is inserted into the vicinity of the distal end of the over tube 3. Here, when the stent 2 is inserted into the over tube 3, as shown in FIG. 3, a part of the side surface of the stent 2 is pressed in a direction of an arrow parallel to the center line C and the stent 2 is deformed with the joint state of the loops 2A held so as to incline the loops 2A about the center line C of the stent 2 by a predetermined angle. Alternatively, the side surfaces located at positions symmetric about the center line C are pressed in a direction parallel to the center line C to deform the loops 2A with slender portions formed therein, as shown in FIG. 4.

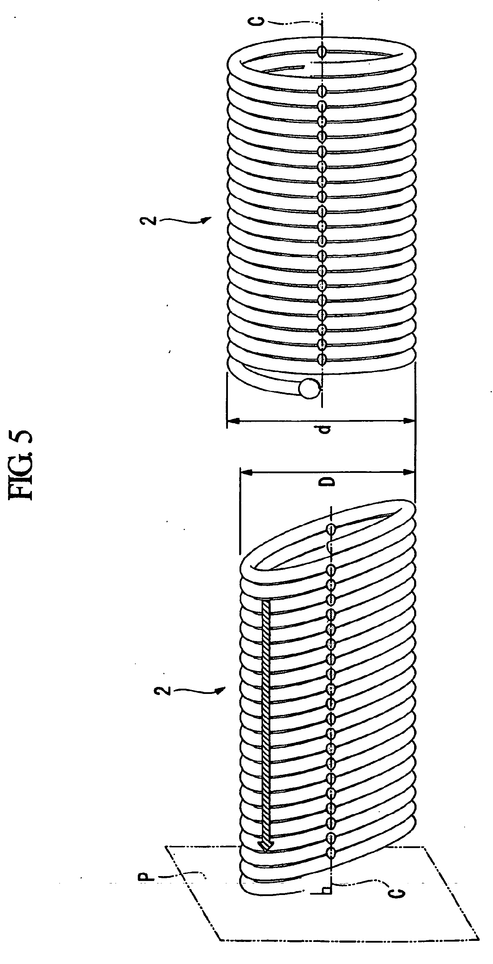

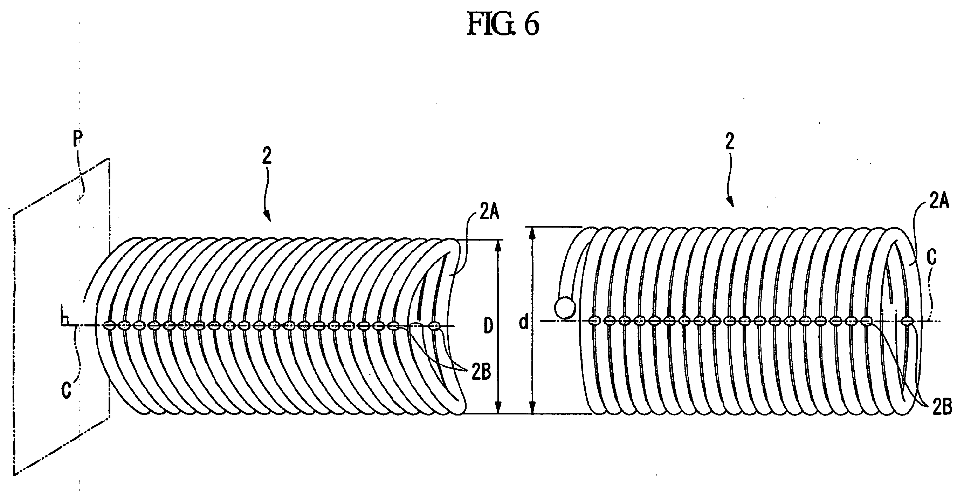

When the stent 2 is deformed as described above, the loop shape is deformed from a ring shape while the jointed state of all the loops 2A is held. As shown in FIGS. 5 and 6, the sectional diameter D of the loops 2A after deformation projected to a virtual plane P perpendicular to the center line C is smaller than the sectional diameter d of the loops 2A before deformation projected to the virtual plane P. Accordingly, the stent 2 can be inserted into the over tube 3.

Subsequently, the over tube 3 into which the stent 2 is inserted is inserted to a predetermined position in a coelom.

At the time of detaining the stent 2, the extrusion member 5 is made to advance to the distal end of the over tube 3. At this time, the extrusion member 5 comes in contact with the end of the stent 2. Then, the stent 2 moves in the over tube 3 by pressing the stent 2 with a force greater than a frictional force between the stent 2 and the over tube 3. Then, the stent 2 is discharged from the over tube 3. At this time, the stent 2 is released from the deformed state in the over tube 3 and is restored to the original shape. Thereafter, by pulling out the over tube 3 from the coelom, the stent 2 is detained at the predetermined position.

In the stent supply instrument 1, by deforming the stent 2 so that the projection area of the stent 2 after deformation onto the virtual plane P is smaller than the projection area of the stent 2 before deformation onto the virtual plane P, it is possible to insert the stent 2 into the over tube 3 with an inner diameter smaller than the outer diameter of the original stent 2. Furthermore, it is possible to insert the stent 2 into the coelom with the deformed state thereof held by the over tube 3. At this time, since the jointed state of the loops 2A of the stent 2 is held in the over tube 3, it is possible to restore the shape of the stent 2 in the coelom by moving the extrusion member 5 to discharge the stent 2 out of the over tube 3 at the time of detaining the stent 2. Accordingly, it is possible to easily supply the resin stent 2 into the coelom.

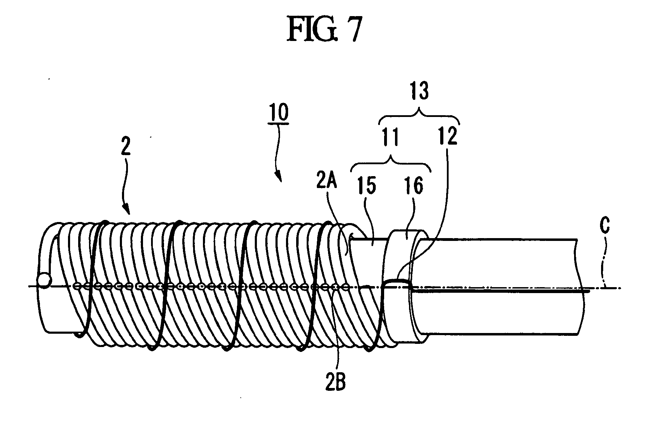

Next, a second embodiment of the invention will be described with reference to FIG. 7.

The same elements as the first embodiment are denoted by the same reference numerals and description thereof is omitted.

The second embodiment is different from the first embodiment, in that a stent supply instrument 10 according to the second embodiment includes a deformation holding section 13 that has an inner diameter holding portion 11 for suppressing the stent 2 from being additionally deformed so as to reduce the projection area of the stent 2 after deformation onto the virtual plane and an outer diameter holding portion 12 for pressing the deformed stent 2 toward the inner diameter holding portion 11.

The inner diameter holding portion 11 includes a main body 15 having a columnar shape and a locking member 16 disposed to protrude in a ring shape from the main body 15 so as to suppress the movement of the stent 2 disposed outside the distal side of the main body 15 toward the proximal end of the main body 15.

The outer diameter holding portion 12 is formed in a line shape and is detachably wound on the stent 2 disposed outside the main body 15. In order to hold the state of the outer diameter holding portion 12 wound on the stent 2, a part of the outer diameter holding portion 12 is detachably adhered to the surface of the locking member 16. Adhesive strengths between the outer diameter holding portion 12 and the stent 2 and between the outer diameter holding portion 12 and the locking member 16 are larger than the force necessary for holding the deformed state of the stent 2 and smaller than the force for drawing the outer diameter holding member 12.

Next, operations of the stent supply instrument 10 according to this embodiment will be described.

First, the stent 2 is inserted into the vicinity of the distal end of the main body 15 of the inner diameter holding portion 11 up to the position at which an end of the stent 2 is locked to the locking member 16. Here, at the time of disposing the stent 2 outside the main body 15, the stent 2 is deformed in the same shape as the first embodiment.

Subsequently, the outer diameter holding portion 12 is wound on the surface of the deformed stent 2. At this time, the outer diameter holding portion 12 is spirally wound from the distal end of the stent 2 to the proximal side thereof to press the stent 2 against the inner diameter holding portion 11.

In this state, the stent supply instrument 10 is inserted to a predetermined position in the coelom.

At the time of detaining the stent 2, the outer diameter holding portion 12 is drawn from the proximal side so as to be separated from the locking member 16. Then, the outer diameter holding portion 12 is further drawn to be separated from the surface of the stent 2. At this time, the pressing force of the outer diameter holding portion 12 against the stent 2 disappears and thus the stent 2 is restored to the shape before deformation. In this state, by pulling out the inner diameter holding portion 11, the stent 2 is detained.

In the stent supply instrument 10, it is possible to obtain the same advantages as the first embodiment. Specifically, since the stent 2 after deformation is also supported from the inside in the diameter direction, it is possible to supply the stent 2 to a predetermined position without further deforming the stent. Furthermore, by moving the outer diameter holding portion 12 to the proximal side relative to the stent 2 and separating the outer diameter holding portion 12 from the stent 2, it is possible to easily restore the shape of the stent 2.

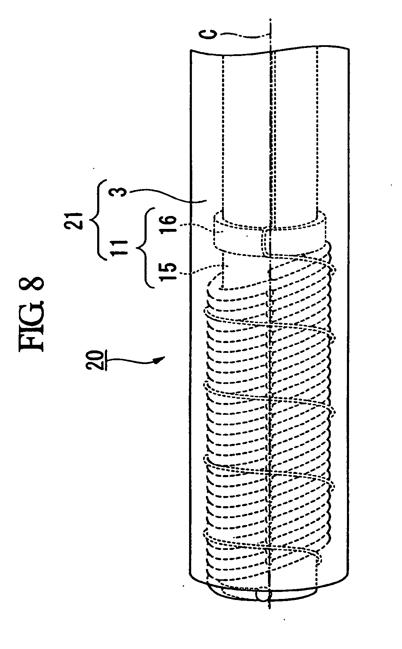

Next, a third embodiment of the invention will be described with reference to FIG. 8.

The same elements as the above-mentioned embodiments are denoted by the same reference numerals and description thereof will be omitted.

The third embodiment is different from the second embodiment, in that a deformation holding section 21 of a stent supply instrument 20 according to the third embodiment includes the over tube 3 according to the first embodiment as the outer diameter holding portion.

Operations of the stent supply instrument 20 will be described.

First, similarly to the second embodiment, the stent 2 is inserted into the vicinity of the distal end of the main body 15 up to the position at which an end of the stent 2 is locked to the locking member 16 of the main body 15 of the inner diameter holding portion 11. At this time, the stent 2 is deformed in the same shape as the first embodiment.

Subsequently, the inner diameter holding portion 11 is inserted into the over tube 3 along with the stent 2. At this time, the stent 2 is pressed against the inner diameter holding portion 11 by the over tube 3.

In this state, the stent supply instrument 20 is inserted into a predetermined position in a coelom.

At the time of detaining the stent 2, the over tube 3 is drawn to the proximal side relative to the inner diameter holding portion 11 to expose the stent 2 to the inside of the coelom. At this time, the pressing force of the over tube 3 against the stent 2 disappears and thus the stent 2 is restored to the shape before deformation. In this state, by pulling out the inner diameter holding portion 11, the stent 2 is detained.

In the stent supply instrument 20, the stent 2 is covered with the over tube 3 by inserting the inner diameter holding portion 11 with the deformed stent 2 disposed on the outer peripheral surface thereof into the over tube 3. Accordingly, the deformed state of the stent 2 can be properly held in the way of the insertion of the stent to the predetermined position.

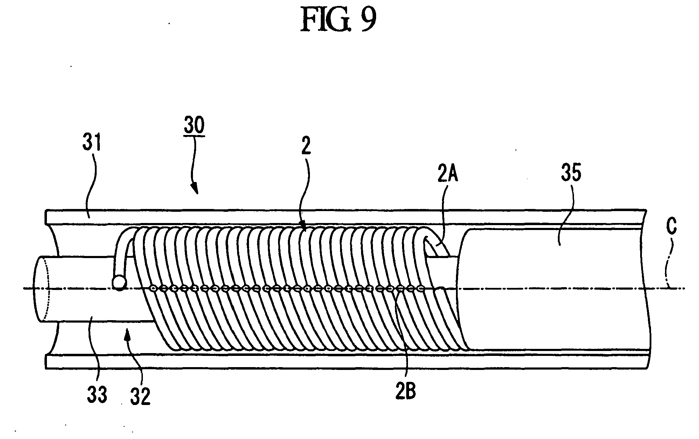

Next, a fourth embodiment of the invention will be described with reference to FIG. 9.

The same elements as the above-mentioned embodiments are denoted by the same reference numerals and description thereof will be omitted.

The fourth embodiment is different from the first embodiment, in that the inner diameter of the deformed stent 2 is kept larger than the outer diameter of an insertion section 33 of an endoscope 32 by means of an over tube 31 of a stent supply instrument 30 according to the fourth embodiment.

The insertion section 33 is inserted into the stent 2 that is deformed and inserted into the over tube 31, so as to freely advance and retreat.

An extrusion member 35 is formed in a substantially cylindrical shape and has such an inner diameter that the insertion section 33 can pass therethrough and such an outer diameter that the extrusion member 35 can come in contact with the end of the stent 2 and move in the over tube 31 so as to advance and retreat.

It is desired that the distal end of the over tube 31 into which the stent 2 is inserted be provided with an observation window for enabling the observation from the insertion section 33.

Next, operations of the stent supply instrument 30 according to this embodiment will be described.

First, similarly to the first embodiment, the stent 2 is inserted into the vicinity of the distal end of the over tube 31 with the stent 2 deformed.

Subsequently, the insertion section 33 of the endoscope 32 is inserted into the over tube 31 into which the stent 2 is inserted. At this time, since the inner diameter of the deformed stent 2 is larger than the outer diameter of the insertion section 33, the insertion section 33 can be made to protrude from the distal end of the over tube 31 therethrough.

The insertion section 33 is inserted into a predetermined position in a coelom and then the over tube 31 is inserted into the coelom.

At the time of detaining the stent 2, the detainment position is observed by the use of the endoscope 32 and then the extrusion member 35 is made to advance to the distal end of the over tube 31. At this time, similarly to the first embodiment, the extrusion member 35 comes in contact with the end of the stent 2, and the stent 2 moves in the over tube 31 and is discharged from the distal end of the over tube 31. Then, the stent 2 is released from the deformed state in the over tube 31, is restored to the original shape, and is detained.

In the stent supply instrument 30, by inserting the insertion section 33 of the endoscope 32 into the over tube 31, it is possible to visually observe the detainment position of the stent 2 and the supply and detainment states of the stent 2 by the use of the endoscope 32.

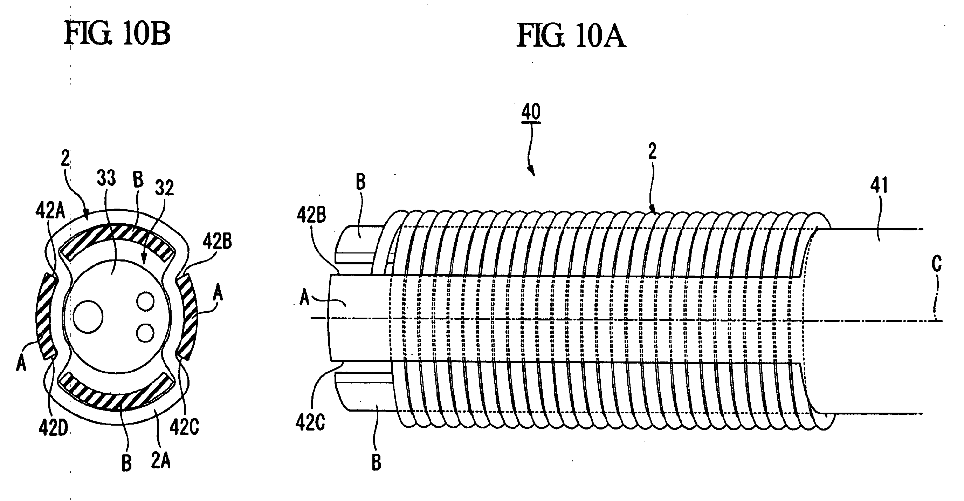

Next, a fifth embodiment of the invention will be described with reference to FIG. 10A and FIG. 10B.

The same elements as the above-mentioned embodiments are denoted by the same reference numerals and description thereof will be omitted.

The fifth embodiment is different from the fourth embodiment, in that an over tube 41 of a stent supply instrument 40 according to the fifth embodiment is formed in a cylindrical shape with an outer diameter smaller than the outer diameter of the stent 2 and a plurality of slits 42A, 42B, 42C, and 42D extending along the center line C is formed in the distal end thereof.

The slits 42A, 42B, 42C, and 42D are disposed at positions where two planes intersecting each other at the center line C of the over tube 41 intersect the over tube 41. The stent 2 engages with the over tube 41 so that it is provided inside the over tube 41 at a region A which is positioned between the slits 42A and 42D and between the slits 42B and 42C and is provided outside the over tube 41 at a region B which is positioned between the slits 42A and 42B and between the slits 42C and 42D. Accordingly, the projection area of the loops 2A after deformation projected onto the virtual plane of the stent 2 is smaller than the projection area of the loops 2A before deformation projected onto the virtual plane.

Next, operations of the stent supply instrument 40 according to this embodiment will be described.

First, the stent 2 is curved and inserted into the slits 42A, 42B, 42C, and 42D of the over tube 41 as described above to lock the stent 2 to the over tube 41.

Subsequently, the insertion section 33 of the endoscope 32 is inserted into the over tube 41 with which the stent 2 engages. Thereafter, the insertion section 33 is inserted into a predetermined position in a coelom and then the over tube 41 is inserted into a position where the distal end of the over tube 41 is located at the same position as the distal end of the insertion section 33.

At the time of detaining the stent 2, the detainment position is observed by the use of the insertion section 33 and then the extrusion member 35 is made to advance toward the distal end of the over tube 41. At this time, similarly to the first embodiment, the extrusion member 35 comes in contact with the end of the stent 2, and the stent 2 moves toward the distal end of the over tube 41 and is separated from the slits 42A, 42B, 42C, and 42D. At this time, the stent 2 is released from the deformed state in which it is locked to the over tube 41, is restored to the original shape, and is detained.

In the stent supply instrument 40, by inserting a part of the stent 2 into the slits 42A, 42B, 42C, and 42D, a part of the side surface of the stent 2 is disposed along the outer peripheral surface of the over tube 41 and the other part thereof is disposed along the inner peripheral surface of the over tube 41. As a result, the stent 2 can be deformed with respect to the outer periphery of the over tube 41 and thus the projection area of the loops 2A after deformation onto the virtual plane can be made smaller than the projection area thereof before deformation.

The technical scope of the invention is not limited to the above-mentioned embodiments, but various modifications such as addition, omission, and replacements of configurations may be made therein.

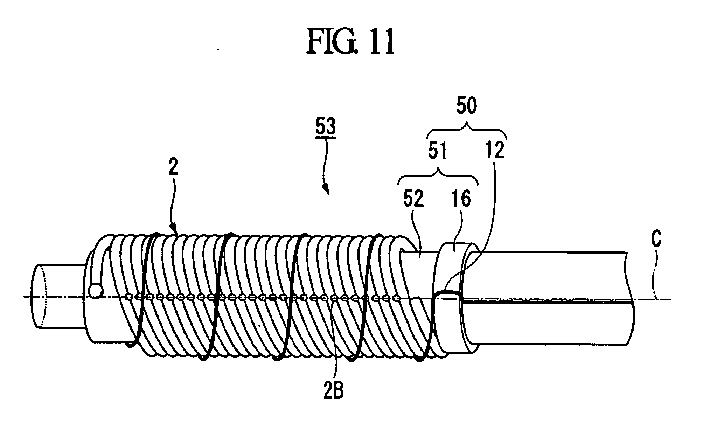

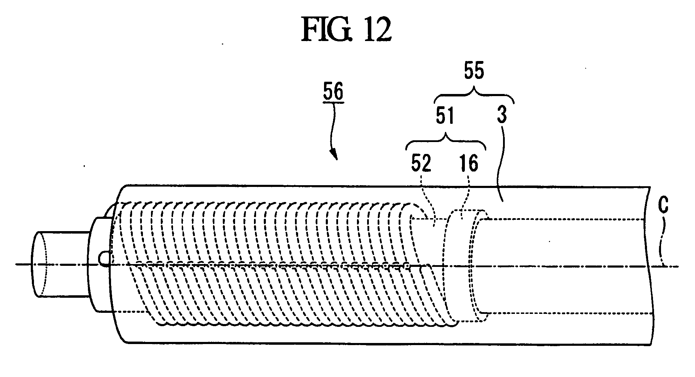

For example, as shown in FIG. 11, a stent supply instrument 53 may be configured in which a main body 52 of an inner diameter holding portion 51 of a deformation holding section 50 is formed in a tube shape, has an inner diameter larger than the outer diameter of the insertion section 33 of the endoscope 32, and allows the insertion section 33 to be inserted therethrough so as to freely advance and retreat, instead of the inner diameter holding portion 11 of the deformation holding section 13 according to the second embodiment. Similarly, as shown in FIG. 12, a stent supply instrument 56 may be configured which includes a deformation holding section 55 having the same inner diameter holding portion 51.

In any case, the same advantages as the fourth embodiment can be obtained.

The invention is not limited to the above-mentioned embodiments, but is limited to only the scope of the appended claims.

## Claims

1. A stent supply instrument (1, 10, 20, 30, 40, 53, 56) comprising:
a plurality of loops (2A) formed by spirally winding a wire or a strand wire made of a resin; and
a deformation holding section (3, 13, 21, 31, 41, 50, 55),
wherein the stent is released from the deformation holding section by means of a relative movement between the deformation holding section and the stent;
**characterized by** further comprising
joint portions (2B) that join each of the plurality of loops to each other along a common center line (C),
wherein the deformation holding section (3, 13, 21, 31, 41, 50, 55) is adapted to hold a deformed shape of a stent (2) detained in a coelom while holding the jointed state of the loops so that a projection area (P) of the loops after deformation projected onto a virtual plane perpendicular to the center axis is smaller than the projection area of the loops before deformation projected onto the virtual plane.

2. The stent supply instrument according to claim 1,
wherein the deformation holding section is formed in a cylindrical shape with an inner diameter smaller than an outer diameter of the stent, and
wherein the stent supply instrument further includes a release portion (5, 35) disposed in the deformation holding section so as to advance and retreat and to come in contact with the stent of which the deformed state is held by the deformation holding section.

3. The stent supply instrument according to claim 1, wherein the deformation holding section includes an inner diameter holding portion (11, 51) for suppressing the stent from being additionally deformed so as to reduce a projection area of the stent after deformation onto the virtual plane and an outer diameter holding portion (12) for pressing the stent after deformation against the inner diameter holding portion.

4. The stent supply instrument according to claim 3, wherein the outer diameter holding portion is formed in a line shape and is wound on the stent.

5. The stent supply instrument according to claim 3, wherein the outer diameter holding portion is formed in a tube shape with an inner diameter smaller than the outer diameter of the stent before deformation and equal to or greater than the outer diameter of the loops after deformation projected onto the virtual plane.

6. The stent supply instrument according to any one of claims 1 to 5, wherein the deformation holding section holds the deformed stent with an inner diameter greater than the outer diameter of an insertion section (33) of an endoscope (32) and allows the insertion section to pass through the stent so as to freely advance and retreat.

7. The stent supply instrument according to claim 1,
wherein the deformation holding section is formed in a cylindrical shape with an outer diameter smaller than the outer diameter of the stent, and
wherein a plurality of slits (42A, 428, 42C, 42D) extending in a center line direction is formed at the distal end of the deformation holding section.

## Patentansprüche

1. Ein Instrument (1, 10 , 20, 30, 40, 53, 56) zum Einführen eines Stents, aufweisend:
eine Mehrzahl von Windungen (2A), gebildet durch spiralförmiges Wickeln einer Litze oder eines verlitzten Drahtes aus einem Harz; und
einen Verformungshalteabschnitt (3, 13, 21, 31, 41, 50, 55),
wobei der Stent von dem Verformungshalteabschnitt durch eine Relativbewegung zwischen Verformungshalteabschnitt und Stent freigegeben wird,
**dadurch gekennzeichnet, dass** es weiterhin aufweist:
Verbindungsabschnitte (2B), welche die Mehrzahl von Windungen entlang einer gemeinsamen Mittellinie (C) aneinanderfügen,
wobei der Verformungshalteabschnitt (3, 13, 21, 31, 41, 50, 55) eine verformte Gestalt eines Stents (2) zu halten vermag, der in einem Körperhohlraum gehalten ist, wobei der aneinandergefügte Zustand der Windungen so gehalten ist, dass eine Projektionsfläche (P) der Windungen nach der Verformung, projiziert auf eine virtuelle Ebene senkrecht zu der Mittelachse, kleiner als die Projektionsfläche der Windungen vor der Verformung, projiziert auf die virtuelle Ebene, ist.

2. Das Instrument zum Einführen eines Stents nach Anspruch 1,
wobei der Verformungshalteabschnitt in Zylinderform ausgebildet ist mit einem Innendurchmesser kleiner als er Außendurchmessser des Stents, und
wobei das Instrument zum Einführen des Stents weiterhin einen Freigabeabschnitt (5, 35) aufweist, der in dem Verformungshalteabschnitt angeordnet ist, um sich vor und zurück zu bewegen und in Kontakt mit dem Stent zu gelangen, dessen verformter Zustand von dem Verformungshalteabschnitt gehalten ist.

3. Das Instrument zum Einführen eines Stents nach Anspruch 1,
wobei der Verformungshalteabschnitt einen Innendurchmesserhalteabschnitt (11, 51), um zu Unterbinden, dass der Stent zusätzlich verformt wird, um eine Projektionsfläche des Stents nach der Verformung auf die virtuelle Ebene zu verkleinern und einen Außendurchmesserhalteabschnitt (12) aufweist, um gegen den Stent nach Verformung gegen den Innendurchmesserhalteabschnitt zu drücken.

4. Das Instrument zum Einführen eines Stents nach Anspruch 3,
wobei der Außendurchmesserhalteabschnitt linienförmig ausgebildet und auf den Stent gewickelt ist.

5. Das Instrument zum Einführen eines Stents nach Anspruch 3,
wobei der Außendurchmesserhalteabschnitt rohrförmig ausgebildet ist, mit einem Innendurchmesser kleiner als der Außendurchmesser des Stents vor der Verformung und gleich oder größer als der Außendurchmesser der Windungen nach der Verformung, projiziert auf die virtuelle Ebene.

6. Das Instrument zum Einführen eines Stents nach einem der Ansprüche 1 bis 5,
wobei der Verformungshalteabschnitt den verformten Stent mit einem Innendurchmesser größer als der Außendurchmesser eines Einführabschnitts (33) eines Endoskops (32) hält und es dem Einführabschnitt ermöglicht, den Stent frei vor und zurück beweglich zu durchtreten.

7. Das Instrument zum Einführen eines Stents nach Anspruch 1,
wobei der Verformungshalteabschnitt in Zylinderform ausgebildet ist, mit einem Außendurchmesser kleiner als der Außendurchmesser des Stents, und
wobei eine Mehrzahl von Schlitzen (42A, 42B, 42C, 42D), die sich in einer Mittellinienrichtung erstrecken, am distalen Ende des Verformungshalteabschnitts ausgebildet ist.

## Revendications

1. Instrument de délivrance d'endoprothèse vasculaire (1, 10, 20, 30, 40, 53, 56) comprenant :
une pluralité de boucles (2A) formées en enroulant en spirale un fil ou un brin composé d'une résine ; et
une section de maintien de déformation (3, 13, 21, 31, 41, 50, 55),
dans lequel l'endoprothèse vasculaire est libérée de la section de maintien de déformation au moyen d'un mouvement relatif entre la section de maintien de déformation et l'endoprothèse vasculaire ;
**caractérisé en ce que** comprenant en outre
des parties de jonction (2B) qui joignent chacune de la pluralité de boucles les unes aux autres le long d'une ligne centrale (C) commune,
dans lequel la section de maintien de déformation (3, 13, 21, 31, 41, 50, 55) est adaptée à maintenir une forme déformée d'une endoprothèse vasculaire (2) contenue dans un coelome tout en maintenant l'état joint des boucles de telle manière qu'une zone de projection (P) des boucles après déformation projetées sur un plan virtuel perpendiculaire à l'axe central est plus petite que la zone de projection des boucles avant déformation projetées sur le plan virtuel.

2. Instrument de délivrance d'endoprothèse vasculaire selon la revendication 1,
dans lequel la section de maintien de déformation est formée à une forme cylindrique avec un diamètre intérieur plus petit qu'un diamètre extérieur de l'endoprothèse vasculaire, et
dans lequel l'instrument de délivrance d'endoprothèse vasculaire inclut en outre une partie de libération (5, 35) disposée dans la section de maintien de déformation de manière à avancer et se rétracter et à venir en contact avec l'endoprothèse vasculaire dont l'état déformé est maintenu par la section de maintien de déformation.

3. Instrument de délivrance d'endoprothèse vasculaire selon la revendication 1, dans lequel la section de maintien de déformation inclut une partie de maintien de diamètre intérieur (11, 51) pour empêcher que l'endoprothèse vasculaire ne soit encore déformée de manière à réduire une zone de projection de l'endoprothèse vasculaire après déformation sur le plan virtuel et une partie de maintien de diamètre extérieur (12) pour presser l'endoprothèse vasculaire après déformation contre la partie de maintien de diamètre intérieur.

4. Instrument de délivrance d'endoprothèse vasculaire selon la revendication 3, dans lequel la partie de maintien de diamètre extérieur est formée à une forme linéaire et est enroulée sur l'endoprothèse vasculaire.

5. Instrument de délivrance d'endoprothèse vasculaire selon la revendication 3, dans lequel la partie de maintien de diamètre extérieur est formée à une forme de tube avec un diamètre intérieur plus petit que le diamètre extérieur de l'endoprothèse vasculaire avant déformation et égal ou supérieur au diamètre extérieur des boucles après déformation projetées sur le plan virtuel.

6. Instrument de délivrance d'endoprothèse vasculaire selon l'une quelconque des revendications 1 à 5, dans lequel la section de maintien de déformation maintient l'endoprothèse vasculaire déformée avec un diamètre intérieur plus grand que le diamètre extérieur d'une section d'insertion (33) d'un endoscope (32) et permet que la section d'insertion passe à travers l'endoprothèse vasculaire de manière à avancer et se rétracter librement.

7. Instrument de délivrance d'endoprothèse vasculaire selon la revendication 1,
dans lequel la section de maintien de déformation est formée à une forme cylindrique avec un diamètre extérieur plus petit que le diamètre extérieur de l'endoprothèse vasculaire, et
dans lequel une pluralité de fentes (42A, 42B, 42C, 42D) s'étendant dans la direction d'une ligne centrale est formée au niveau de l'extrémité distale de la section de maintien de déformation.
